# EUROPEAN PATENT APPLICATION

(11) **EP 4 218 816 A2**
(43) Date of publication of application: **02.08.2023**
(21) Application number: 23157462.5
(22) Date of filing: 31.03.2015
(51) Int. Cl.: A61K 39/395, A61P 7/04, C07K 16/36, C07K 16/46

(54) **PHARMACEUTICAL COMPOSITION FOR USE IN PREVENTION AND/OR TREATMENT OF DISEASE THAT DEVELOPS OR PROGRESSES AS A RESULT OF DECREASE OR LOSS OF ACTIVITY OF BLOOD COAGULATION FACTOR VIII AND/OR ACTIVATED BLOOD COAGULATION FACTOR VIII**

(30) Priority: 20.06.2014 JP 2014127240; 07.11.2014 JP 2014226988
(62) Divisional of application: 15809216.3
(71) Applicant: Chugai Seiyaku Kabushiki Kaisha, Kita-ku Tokyo 115-8543 (JP)
(72) Inventor: YONEYAMA, Koichiro, Tokyo, 103-8324 (JP)
(74) Representative: Vossius & Partner Patentanwälte Rechtsanwälte mbB

(57) **Abstract**

The inventors discovered that by administering a pharmaceutical composition comprising a bispecific antigen-binding molecule that recognizes blood coagulation factor IX and/or activated blood coagulation factor IX and blood coagulation factor X and/or activated blood coagulation factor X according to a given dosage regimen, diseases that develop and/or progress due to a decrease or deficiency in the activity of blood coagulation factor VIII and/or activated blood coagulation factor VIII can be prevented and/or treated more effectively.

## Description

### Technical Field

The present invention relates to a pharmaceutical composition or a dosage regimen used for preventing and/or treating a disease that develops and/or progresses due to a decrease or deficiency in the activity of blood coagulation factor VIII and/or activated blood coagulation factor VIII. More specifically, the present invention relates to a dosage regimen or a pharmaceutical composition comprising a bispecific antigen-binding molecule that recognizes blood coagulation factor IX and/or activated blood coagulation factor IX and blood coagulation factor X and/or activated blood coagulation factor X, which are used for preventing and/or treating hemophilia A, acquired hemophilia A, von Willebrand disease, and hemophilia A in which an inhibitor against blood coagulation factor VIII and/or activated blood coagulation factor VIII emerges. Furthermore, the present invention relates to a product that includes a statement regarding administration of the pharmaceutical composition comprising the bispecific antigen-binding molecule.

### Background Art

Hemophilia is a hemorrhagic disease caused by a congenital deficiency or dysfunction of coagulation factor VIII (FVIII) or coagulation factor IX (FIX). The former is called hemophilia A and the latter is called hemophilia B. Genes for both factors are located on the X chromosome, and since genetic defects take the X-chromosome-linked recessive hereditary form, 99% or more of the patients who develop the disease are men. It is known that the prevalence rate is approximately one in 10,000 live male births, and the ratio between hemophilia A and hemophilia B is approximately 5:1.

The severity of hemophilia A is defined by the FVIII activity in blood. Patients with an activity of less than 1% are classified as severe, patients with an activity of 1% or more to less than 5% are classified as moderate, and patients with an activity of 5% or more and less than 40% are classified as mild. Severe patients who account for approximately half of hemophilia A patients exhibit bleeding symptoms several times a month, and this frequency is markedly high as compared to those of moderate and mild patients.

For bleeding in hemophilia A patients, FVIII formulations are generally administered (on-demand therapy). In recent years, FVIII formulations are also administered prophylactically to prevent bleeding events (regular replacement therapy; Non-patent Documents 1 and 2). The half-life of FVIII formulations in blood is approximately 8 to 12 hours. Therefore, for continuous prevention, FVIII formulations are administered to patients three times a week (Non-patent Documents 3 and 4). In on-demand therapy, FVIII formulations are also additionally administered at regular intervals as necessary to prevent rebleeding. In addition, FVIII formulations are mainly administered at home, but since they are administered intravenously, the difficulty of securing a blood vessel is a problem. Therefore, there has been a strong need for pharmaceutical agents with a lesser burden regarding their administration as compared to FVIII formulations.

Occasionally, antibodies against FVIII (inhibitors) develop in hemophilia A patients. Such inhibitors counteract the effects of the FVIII formulations. For bleeding in patients who have developed inhibitors (inhibitor patients), bypassing agents are administered. Their mechanisms of action are not dependent on FVIII function, that is, on the function of catalyzing the activation of blood coagulation factor X (FX) by activated blood coagulation factor IX (FIXa). Therefore, in some cases, bypassing agents cannot sufficiently stop the bleeding. Recently, results suggesting the effectiveness of regular administration therapy of bypassing agents have been obtained, but this has not yielded a sufficient effect to suppress bleeding as compared to FVIII formulations. Accordingly, there has been a strong need for therapeutic agents that can be administered subcutaneously, as well as long-acting therapeutic agents that can be administered less frequently, regardless of the presence of inhibitors.

Recently, as a means for solving the problem, antibodies that functionally substitute for FVIII and their use were disclosed (Patent Documents 1, 2, 3, and 4, and Non-patent Documents 5, 6, and 7).

### Prior Art Documents

### [Patent Documents]

[Patent Document 1] WO 2005/035754
[Patent Document 2] WO 2005/035756
[Patent Document 3] WO 2006/109592
[Patent Document 4] WO 2012/067176

### [Non-patent Documents]

[Non-patent Document 1] Blood 58, 1-13 (1981)
[Non-patent Document 2] Nature 312, 330-337 (1984)
[Non-patent Document 3] Nature 312, 337-342 (1984)
[Non-patent Document 4] Biochim.Biophys.Acta 871, 268-278 (1986)
[Non-patent Document 5] Nature Medicine 18, 1570-1574(2012)
[Non-patent Document 6] PLOS ONE 8, 1-13(2013)
[Non-patent Document 7] J Thromb Haemost. 12, 206-213(2014)

### Summary of the Invention

### [Problems to be solved by the Invention]

An objective of the present invention is to provide a more effective pharmaceutical composition or a dosage regimen for preventing and/or treating a disease that develops and/or progresses due to a decrease or deficiency in the activity of blood coagulation factor VIII and/or activated blood coagulation factor VIII.

### [Means for Solving the Problems]

As a result of dedicated research, the present inventors succeeded in discovering a more effective dosage regimen for a pharmaceutical composition containing a bispecific antigen-binding molecule that recognizes blood coagulation factor IX and/or activated blood coagulation factor IX and blood coagulation factor X and/or activated blood coagulation factor X for preventing and/or treating a disease that develops and/or progresses due to a decrease or deficiency in the activity of blood coagulation factor VIII and/or activated blood coagulation factor VIII.

Specifically, the present invention relates to a pharmaceutical composition or a dosage regimen used for preventing and/or treating a disease that develops and/or progresses due to a decrease or deficiency in the activity of blood coagulation factor VIII and/or activated blood coagulation factor VIII, and specifically relates to the following:
[1] a pharmaceutical composition for use in preventing and/or treating a disease that develops and/or progresses due to a decrease or deficiency in the activity of blood coagulation factor VIII and/or activated blood coagulation factor VIII, wherein the composition comprises a bispecific antigen-binding molecule that recognizes blood coagulation factor IX and/or activated blood coagulation factor IX and blood coagulation factor X and/or activated blood coagulation factor X, wherein the antigen-binding molecule is administered at an initial dose of approximately 0.001 to 100 mg/kg and is continually administered several times at continued doses that are nearly the same as or less than the initial dose, wherein the interval between individual administrations is at least one day or longer;
[2] the pharmaceutical composition of [1], wherein the continued dose is a dose selected from the group consisting of the same dose as the initial dose, and approximately one-half, approximately one-third, approximately 0.3-times, approximately one-fourth, approximately one-fifth, and approximately one-tenth the initial dose;
[3] the pharmaceutical composition of [2], wherein the initial dose is 1 mg/kg and at least one of the continued doses is 0.3 mg/kg;
[4] the pharmaceutical composition of [2], wherein the initial dose is 3 mg/kg and at least one of the continued doses is 1 mg/kg;
[5] the pharmaceutical composition of [2], wherein the initial dose is 3 mg/kg and at least one of the continued doses is 3 mg/kg;
[6] the pharmaceutical composition of any one of [1] to [5], wherein at least one of the intervals between individual administrations is one week;
[7] the pharmaceutical composition of any one of [1] to [6], wherein the disease is selected from the group consisting of hemophilia A, acquired hemophilia A, von Willebrand disease, and hemophilia A with emergence of an inhibitor against blood coagulation factor VIII and/or activated blood coagulation factor VIII;
[8] the pharmaceutical composition of any one of [1] to [7], wherein the antigen-binding molecule is the bispecific antibody described below, wherein the first polypeptide and the third polypeptide are associated and the second polypeptide and the fourth polypeptide are associated:
   a bispecific antibody comprising a first polypeptide which is an H chain comprising the amino acid sequence of SEQ ID NO: 20; a second polypeptide which is an H chain comprising the amino acid sequence of SEQ ID NO: 25; a third polypeptide and a fourth polypeptide which are a commonly shared L chain comprising the amino acid sequence of SEQ ID NO: 32;
[9] the pharmaceutical composition of any one of [1] to [8], wherein the antigen-binding molecule is administered subcutaneously;
[10] a product comprising (i) a container; (ii) a pharmaceutical composition in the container, wherein the composition comprises a bispecific antigen-binding molecule that recognizes blood coagulation factor IX and/or activated blood coagulation factor IX and blood coagulation factor X and/or activated blood coagulation factor X; and (iii) a document instructing administration of the antigen-binding molecule at an initial dose of approximately 0.001 to 100 mg/kg and several continued administrations of continued doses that are nearly the same as or less than the initial dose, wherein the interval between individual administrations is at least one day or longer;
[11] the product of [10], wherein the initial dose is 1 mg/kg and at least one of the continued doses is 0.3 mg/kg;
[12] the product of [10] or [11], wherein at least one of the intervals between individual administrations is one week;
[13] the product of any one of [10] to [12], which further comprises a label attached to the container which indicates that the pharmaceutical composition can be used for preventing and/or treating a disease that develops and/or progresses due to a decrease or deficiency in the activity of blood coagulation factor VIII and/or activated blood coagulation factor VIII;
[14] the product of [13], wherein the label indicates that the pharmaceutical composition can be used for the treatment of hemophilia A, acquired hemophilia A, von Willebrand disease, and hemophilia A with emergence of an inhibitor against blood coagulation factor VIII and/or activated blood coagulation factor VIII; and
[15] the product of any one of [10] to [14], wherein the antigen-binding molecule is the bispecific antibody described below, wherein the first polypeptide and the third polypeptide are associated and the second polypeptide and the fourth polypeptide are associated:
   a bispecific antibody comprising a first polypeptide which is an H chain comprising the amino acid sequence of SEQ ID NO: 20; a second polypeptide which is an H chain comprising the amino acid sequence of SEQ ID NO: 25; and a third polypeptide and a fourth polypeptide which are commonly shared L chain comprising the amino acid sequence of SEQ ID NO: 32.

Furthermore, the present invention relates to:
[16] a method for preventing and/or treating a disease that develops and/or progresses due to a decrease or deficiency in the activity of blood coagulation factor VIII and/or activated blood coagulation factor VIII, which comprises administering a bispecific antigen-binding molecule which recognizes blood coagulation factor IX and/or activated blood coagulation factor IX and blood coagulation factor X and/or activated blood coagulation factor X at an initial dose of approximately 0.001 to 100 mg/kg and continually administering the antigen-binding molecule several times at continued doses that are nearly the same as or less than the initial dose, wherein the interval between administrations is at least one day or longer;
[17] an antigen-binding molecule for use in preventing and/or treating a disease that develops and/or progresses due to a decrease or deficiency in the activity of blood coagulation factor VIII and/or activated blood coagulation factor VIII, wherein the bispecific antigen-binding molecule recognizes blood coagulation factor IX and/or activated blood coagulation factor IX and blood coagulation factor X and/or activated blood coagulation factor X and is administered at an initial dose of approximately 0.001 to 100 mg/kg and then continually administered several times at continued doses that are nearly the same as or less than the initial dose, wherein the interval between administrations is at least one day or longer; and
[18] use of an antigen-binding molecule in the manufacture of a pharmaceutical composition used for preventing and/or treating a disease that develops and/or progresses due to a decrease or deficiency in the activity of blood coagulation factor VIII and/or activated blood coagulation factor VIII, wherein the bispecific antigen-binding molecule recognizes blood coagulation factor IX and/or activated blood coagulation factor IX and blood coagulation factor X and/or activated blood coagulation factor X and is administered at an initial dose of approximately 0.001 to 100 mg/kg and then continually administered several times at continued doses that are nearly the same as or less than the initial dose, wherein the interval between administrations is at least one day or longer.

### Brief Description of the Drawings

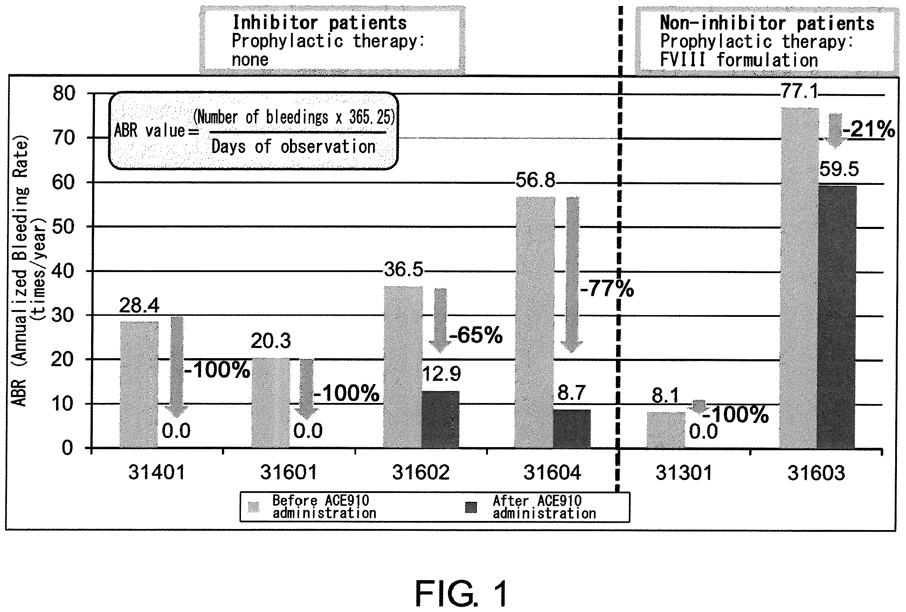

Fig. 1 shows a graph indicating the changes in bleeding frequency of inhibitor-possessing patients and inhibitor-non-possessing patients who received ACE910 administration for 12 weeks in Examination 1. The numbers on the horizontal axis indicate the numbering of the patients who participated in the examination.

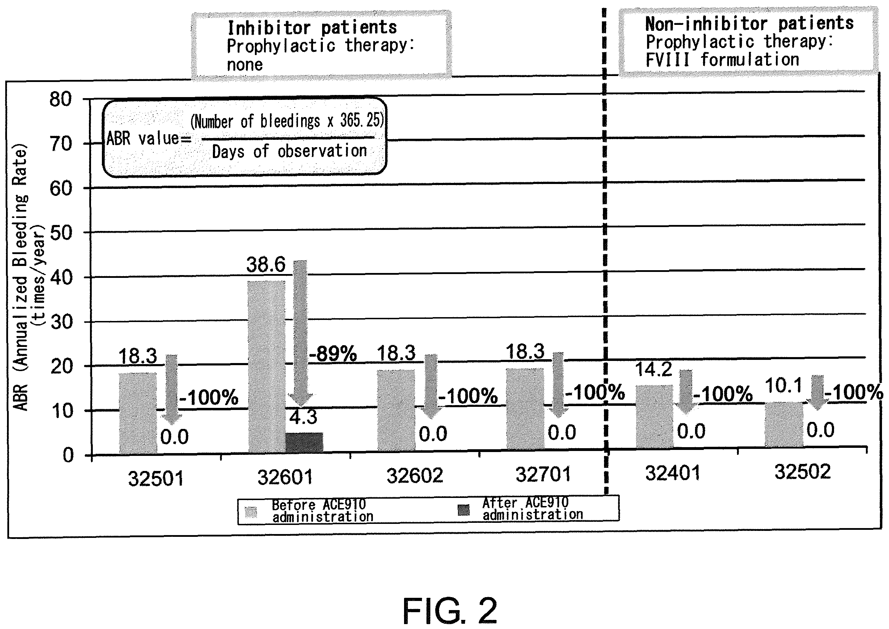

Fig. 2 shows a graph indicating the changes in bleeding frequency of inhibitor-possessing patients and inhibitor-non-possessing patients who received ACE910 administration for 12 weeks in Examination 2. The numbers on the horizontal axis indicate the numbering of the patients who participated in the examination.

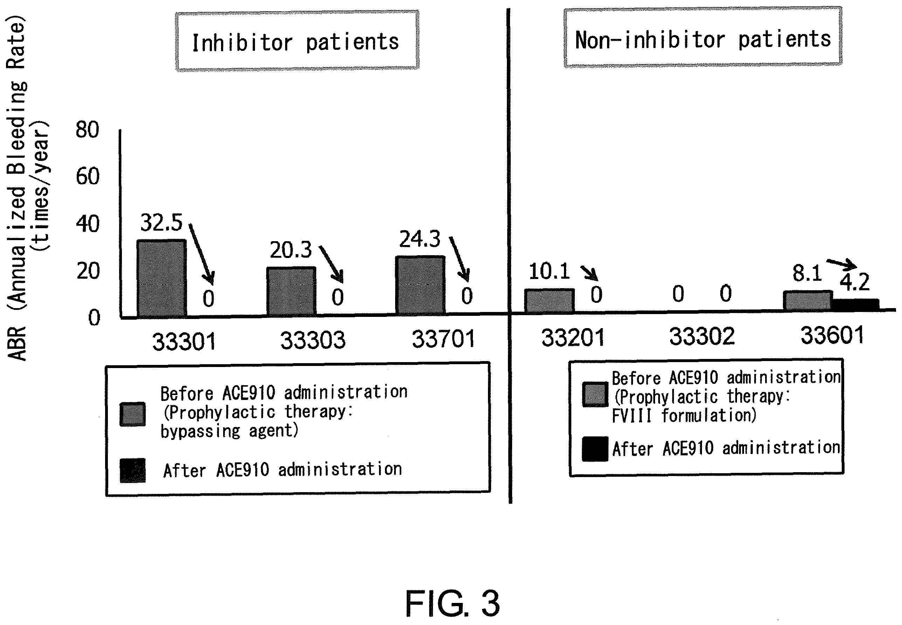

Fig. 3 shows a graph indicating the changes in bleeding frequency of inhibitor-possessing patients and inhibitor-non-possessing patients who received ACE910 administration for 12 weeks in Examination 3. The numbers on the horizontal axis indicate the numbering of the patients who participated in the examination.

### Mode for Carrying Out the Invention

A bispecific antigen-binding molecule that recognizes blood coagulation factor IX and/or activated blood coagulation factor IX and blood coagulation factor X and/or activated blood coagulation factor X preferably has an activity of functionally substituting for coagulation factor VIII.

In the present invention, the phrase "functionally substitute for coagulation factor VIII" means that coagulation factor IX (FIX) or coagulation factor IXa (FIXa), and coagulation factor X (FX) are recognized, and the activation of FX by FIXa is promoted (FXa generation by FIXa is promoted). FXa generation-promoting activity can be evaluated using, for example, a measurement system comprising FXIa, FX, the synthetic substrate S-2222 (a synthetic substrate of FXa), and phospholipids. Such a measurement system shows the correlation between the severity of the disease and the clinical symptoms in hemophilia A cases (Rosen S, Andersson M, Blomback M et al. Clinical applications of a chromogenic substrate method for determination of FVIII activity. Thromb Haemost 1985; 54: 811-23).

Such antigen-binding molecules can be obtained according to methods described, for example, in WO2005/035756, WO2006/109592, and WO2012/067176. Specifically, based on the sequences of antibodies against coagulation factor IX and/or activated coagulation factor IX and antibodies against coagulation factor X, antibodies can be generated using genetic recombination techniques known to those skilled in the art. A polynucleotide encoding an antibody can be constructed based on the sequences of the antibodies against coagulation factor IX and/or activated coagulation factor IX and antibodies against coagulation factor X, and this can be inserted into an expression vector and subsequently expressed in appropriate host cells (see for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

Such bispecific antigen-binding molecules can be isolated from inside host cells or from outside the cells (such as from the medium), and purified as substantially pure and homogeneous antibodies. Isolation and purification of antibodies can be carried out using methods generally used for isolating and purifying antibodies, and are not limited. For example, antibodies can be isolated and purified by appropriately selecting and combining column chromatography columns, filters, ultrafiltration, salting-out, solvent precipitation, solvent extraction, distillation, immunoprecipitation, SDS-polyacrylamide gel electrophoresis, isoelectric focusing, dialysis, recrystallization, and such.

The bispecific antigen-binding molecules of the present invention include the antibodies described, for example, in WO2005/035756, WO2006/109592, and WO2012/067176.

A bispecific antigen-binding molecule comprises a first antigen-binding site and a second antigen-binding site which can specifically bind to at least two different types of antigens. While the first antigen-binding site and the second antigen-binding site of the bispecific antigen-binding molecule of the present invention are not particularly limited as long as they have an activity to bind to coagulation factor IX and/or activated coagulation factor IX, and coagulation factor X, respectively, examples include sites necessary for binding with antigens, such as antibodies, scaffold molecules (antibody-like molecules), and peptides, and fragments containing such sites. A scaffold molecule is a molecule that exhibits a function by binding to a target molecule, and any polypeptide may be used as long as it is a conformationally stable polypeptide that can bind to at least one target antigen. Examples of such polypeptides include antibody variable regions, fibronectin (WO 2002/032925), protein A domain (WO 1995/001937), LDL receptor A domain (WO 2004/044011, WO 2005/040229), ankyrin (WO 2002/020565), as well as the molecules described in Nygren et al. (Current Opinion in Structural Biology, 7: 463-469 (1997); and Journal of Immunol Methods, 290: 3-28 (2004)), Binz et al. (Nature Biotech 23: 1257-1266 (2005)), and Hosse et al. (Protein Science 15: 14-27(2006)). Furthermore, as described in Curr Opin Mol Ther. 2010 Aug; 12(4): 487-95 and Drugs. 2008; 68(7): 901-12, peptide molecules that can bind to the target antigens can also be used.

Bispecific antigen-binding molecules can be produced using, for example, genetic recombination techniques (see, for example, Borrebaeck CAK and Larrick JW, THERAPEUTIC MONOCLONAL ANTIBODIES, Published in the United Kingdom by MACMILLAN PUBLISHERS LTD, 1990). Recombinant antibodies can be obtained by cloning DNAs encoding the antibodies from hybridomas or antibody-producing cells, such as sensitized lymphocytes that produce antibodies, inserting them into suitable vectors, and then introducing them into hosts (host cells) to produce the antibodies.

Furthermore, bispecific antigen-binding molecules may include not only whole antibodies but also antibody fragments and low-molecular-weight antibodies, and modified antibodies.

For example, antibody fragments and low-molecular-weight antibodies include diabodies (Dbs), linear antibodies, and single chain antibody (hereinafter, also denoted as scFv) molecules. Herein, an "Fv" fragment is a smallest antibody fragment and comprises a full antigen recognition site and binding site.

Bispecific antibodies include human antibodies, mouse antibodies, rat antibodies, and such, and their origins are not limited. They may also be genetically modified antibodies, such as chimeric antibodies and humanized antibodies.

Methods for obtaining human antibodies are already known. For example, a human antibody of interest can be obtained by immunizing a transgenic animal carrying the entire repertoire of human antibody genes with an antigen of interest (see International Publication No. WO 93/12227, WO 92/03918, WO 94/02602, WO 94/25585, WO 96/34096, and WO 96/33735).

Genetically modified antibodies can also be produced using known methods. Specifically, for example, a chimeric antibody is an antibody that comprises H chain and L chain variable regions of an immunized animal antibody, and H chain and L chain constant regions of a human antibody. Chimeric antibodies can be obtained by linking DNAs encoding the variable regions of an antibody derived from an immunized animal with DNAs encoding the constant regions of a human antibody, inserting this into an expression vector, and then introducing this into host cells to produce the antibodies.

A humanized antibody is a modified antibody which is also often referred to as a reshaped human antibody. A humanized antibody is constructed by transferring the CDRs of an antibody derived from an immunized animal to the complementarity determining regions of a human antibody. General genetic recombination techniques for producing them are also known (see European Patent Application Publication No. EP 239400; International Publication No. WO 96/02576; Sato K et al., Cancer Research 1993, 53: 851-856; International Publication No. WO 99/51743).

More specifically, the bispecific antigen-binding molecule of the present invention is a bispecific antibody in which a first polypeptide and a third polypeptide are associated and a second polypeptide and a fourth polypeptide are associated, and is preferably any one of the antibodies described below:
(a) a bispecific antibody comprising a first polypeptide which is an H chain containing the amino acid sequence of SEQ ID NO: 1; a second polypeptide which is an H chain containing the amino acid sequence of SEQ ID NO: 4; and a third and fourth polypeptide which are a commonly shared L chain containing the amino acid sequence of SEQ ID NO: 9 (Q1 -G4k/J268-G4h/L45-k);
(b) a bispecific antibody comprising a first polypeptide which is an H chain containing the amino acid sequence of SEQ ID NO: 1; a second polypeptide which is an H chain containing the amino acid sequence of SEQ ID NO: 5; and a third and fourth polypeptide which are a commonly shared L chain containing the amino acid sequence of SEQ ID NO: 9 (Ql-G4k/J321-G4h/L45-k);
(c) a bispecific antibody comprising a first polypeptide which is an H chain containing the amino acid sequence of SEQ ID NO: 2; a second polypeptide which is an H chain containing the amino acid sequence of SEQ ID NO: 6; and a third and fourth polypeptide which are a commonly shared L chain containing the amino acid sequence of SEQ ID NO: 8 (Q31-z7/J326-z107/L2-k);
(d) a bispecific antibody comprising a first polypeptide which is an H chain containing the amino acid sequence of SEQ ID NO: 3; a second polypeptide which is an H chain containing the amino acid sequence of SEQ ID NO: 7; and a third and fourth polypeptide which are a commonly shared L chain containing the amino acid sequence of SEQ ID NO: 9 (Q64-z55/J344-z107/L45-k);
(e) a bispecific antibody comprising a first polypeptide which is an H chain containing the amino acid sequence of SEQ ID NO: 10; a second polypeptide which is an H chain containing the amino acid sequence of SEQ ID NO: 6; and a third and fourth polypeptide which are a commonly shared L chain containing the amino acid sequence of SEQ ID NO: 30 (Q64-z7/J326-z107/L334-k);
(f) a bispecific antibody comprising a first polypeptide which is an H chain containing the amino acid sequence of SEQ ID NO: 10; a second polypeptide which is an H chain containing the amino acid sequence of SEQ ID NO: 7; and a third and fourth polypeptide which are a commonly shared L chain containing the amino acid sequence of SEQ ID NO: 33 (Q64-z7/J344-z107/L406-k);
(g) a bispecific antibody comprising a first polypeptide which is an H chain containing the amino acid sequence of SEQ ID NO: 11; a second polypeptide which is an H chain containing the amino acid sequence of SEQ ID NO: 4; and a third and fourth polypeptide which are a commonly shared L chain containing the amino acid sequence of SEQ ID NO: 33 (Q85-G4k/J268-G4h/L406-k);
(h) a bispecific antibody comprising a first polypeptide which is an H chain containing the amino acid sequence of SEQ ID NO: 11; a second polypeptide which is an H chain containing the amino acid sequence of SEQ ID NO: 5; and a third and fourth polypeptide which are a commonly shared L chain containing the amino acid sequence of SEQ ID NO: 30 (Q85-G4k/J321-G4h/L334-k);
(i) a bispecific antibody comprising a first polypeptide which is an H chain containing the amino acid sequence of SEQ ID NO: 12; a second polypeptide which is an H chain containing the amino acid sequence of SEQ ID NO: 21; and a third and fourth polypeptide which are a commonly shared L chain containing the amino acid sequence of SEQ ID NO: 33 (Q153-G4k/J232-G4h/L406-k);
(j) a bispecific antibody comprising a first polypeptide which is an H chain containing the amino acid sequence of SEQ ID NO: 13; a second polypeptide which is an H chain containing the amino acid sequence of SEQ ID NO: 22; and a third and fourth polypeptide which are a commonly shared L chain containing the amino acid sequence of SEQ ID NO: 29 (Q354-z106/J259-z107/L324-k);
(k) a bispecific antibody comprising a first polypeptide which is an H chain containing the amino acid sequence of SEQ ID NO: 14; a second polypeptide which is an H chain containing the amino acid sequence of SEQ ID NO: 21; and a third and fourth polypeptide which are a commonly shared L chain containing the amino acid sequence of SEQ ID NO: 33 (Q360-G4k/J232-G4h/L406-k);
(l) a bispecific antibody comprising a first polypeptide which is an H chain containing the amino acid sequence of SEQ ID NO: 15; a second polypeptide which is an H chain containing the amino acid sequence of SEQ ID NO: 23; and a third and fourth polypeptide which are a commonly shared L chain containing the amino acid sequence of SEQ ID NO: 30 (Q360-z 118/J300-z 107/L334-k);
(m) a bispecific antibody comprising a first polypeptide which is an H chain containing the amino acid sequence of SEQ ID NO: 16; a second polypeptide which is an H chain containing the amino acid sequence of SEQ ID NO: 21; and a third and fourth polypeptide which are a commonly shared L chain containing the amino acid sequence of SEQ ID NO: 28 (Q405-G4k/J232-G4h/L248-k);
(n) a bispecific antibody comprising a first polypeptide which is an H chain containing the amino acid sequence of SEQ ID NO: 17; a second polypeptide which is an H chain containing the amino acid sequence of SEQ ID NO: 27; and a third and fourth polypeptide which are a commonly shared L chain containing the amino acid sequence of SEQ ID NO: 34 (Q458-z106/J346-z107/L408-k);
(o) a bispecific antibody comprising a first polypeptide which is an H chain containing the amino acid sequence of SEQ ID NO: 18; a second polypeptide which is an H chain containing the amino acid sequence of SEQ ID NO: 25; and a third and fourth polypeptide which are a commonly shared L chain containing the amino acid sequence of SEQ ID NO: 30 (Q460-z121/J327-z119/L334-k);
(p) a bispecific antibody comprising a first polypeptide which is an H chain containing the amino acid sequence of SEQ ID NO: 19; a second polypeptide which is an H chain containing the amino acid sequence of SEQ ID NO: 24; and a third and fourth polypeptide which are a commonly shared L chain containing the amino acid sequence of SEQ ID NO: 30 (Q499-z 118/J327-z 107/L3 34-k);
(q) a bispecific antibody comprising a first polypeptide which is an H chain containing the amino acid sequence of SEQ ID NO: 19; a second polypeptide which is an H chain containing the amino acid sequence of SEQ ID NO: 24; and a third and fourth polypeptide which are a commonly shared L chain containing the amino acid sequence of SEQ ID NO: 31 (Q499-z118/J327-z107/L377-k);
(r) a bispecific antibody comprising a first polypeptide which is an H chain containing the amino acid sequence of SEQ ID NO: 19; a second polypeptide which is an H chain containing the amino acid sequence of SEQ ID NO: 27; and a third and fourth polypeptide which are a commonly shared L chain containing the amino acid sequence of SEQ ID NO: 28 (Q499-z118/J346-z107/L248-k);
(s) a bispecific antibody comprising a first polypeptide which is an H chain containing the amino acid sequence of SEQ ID NO: 20; a second polypeptide which is an H chain containing the amino acid sequence of SEQ ID NO: 25; and a third and fourth polypeptide which are a commonly shared L chain containing the amino acid sequence of SEQ ID NO: 32 (Q499-z121/J327-z119/L404-k);
(t) a bispecific antibody comprising a first polypeptide which is an H chain containing the amino acid sequence of SEQ ID NO: 20; a second polypeptide which is an H chain containing the amino acid sequence of SEQ ID NO: 26; and a third and fourth polypeptide which are a commonly shared L chain containing the amino acid sequence of SEQ ID NO: 31 (Q499-z121/J339-z119/L377-k); and
(u) a bispecific antibody comprising a first polypeptide which is an H chain containing the amino acid sequence of SEQ ID NO: 12; a second polypeptide which is an H chain containing the amino acid sequence of SEQ ID NO: 35; and a third and fourth polypeptide which are a commonly shared L chain containing the amino acid sequence of SEQ ID NO: 36 (Q153-G4k/J142-G4h/L180-k).

The bispecific antibody of (s) is particularly preferred.

Pharmaceutical compositions of the present invention which are used for therapeutic or preventive purposes can be prepared by mixing, if necessary, with suitable pharmaceutically acceptable carriers, vehicles, and such and made into a freeze-dry formulation or a solution formulation. Examples of suitable pharmaceutically acceptable carriers and vehicles include sterilized water, physiological saline, stabilizers, excipients, antioxidants (such as ascorbic acid), buffers (such as phosphate, citrate, histidine, and other organic acids), antiseptics, surfactants (such as PEG and Tween), chelating agents (such as EDTA), and binders. They may also contain other low-molecular-weight polypeptides, proteins such as serum albumin, gelatin, and immunoglobulins, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol. When preparing an aqueous solution for injection, for example, physiological saline and isotonic solutions containing glucose and other adjuvants such as D-sorbitol, D-mannose, D-mannitol, and sodium chloride may be used, and appropriate solubilizers such as alcohol (for example, ethanol), polyalcohols (such as propylene glycol and PEG), and nonionic surfactants (such as polysorbate 80, polysorbate 20, poloxamer 188, and HCO-50) may be used in combination. By mixing hyaluronidase into the formulation, a larger fluid volume can be administered subcutaneously (Expert Opin Drug Deliv. 2007 Jul; 4(4): 427-40). Further, the pharmaceutical compositions of the present invention may be preliminarily loaded in a syringe. Meanwhile, the solution formulation can be prepared according to the method described in WO 2011/090088.

If necessary, the antigen-binding molecules of the present invention can be encapsulated in microcapsules (e.g., those made of hydroxymethylcellulose, gelatin, and poly(methylmethacrylate)), or prepared as colloidal drug delivery systems (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the antigen-binding molecules of the present invention (Langer et al., J. Biomed. Mater. Res. 15: 267-277 (1981); Langer, Chemtech. 12: 98-105 (1982); U.S. Patent No. 3,773,919; European Patent Application Publication No. EP 58,481; Sidman et al., Biopolymers 22: 547-556 (1983); EP 133,988).

The pharmaceutical compositions of the present invention can be administered to a patient *via* any appropriate route, for example, intravenously by bolus injection or continuous infusion for a given period, intramuscularly, intraperitoneally, intracerebrospinally, transdermally, subcutaneously, intraarticularly, sublingually, intrasynovially, orally, by inhalation, locally, or externally. Intravenous administration or subcutaneous administration is preferred.

In the present invention, "initial dose" refers to, for example, the dose used when a bispecific antigen-binding molecule of the present invention is administered to a patient for the first time. The initial dose is within the range of approximately 0.001 mg/kg to approximately 100 mg/kg, and is for example, approximately 0.001 mg/kg, approximately 0.002 mg/kg, approximately 0.0025 mg/kg, approximately 0.003 mg/kg, approximately 0.004 mg/kg, approximately 0.005 mg/kg, approximately 0.006 mg/kg, approximately 0.007 mg/kg, approximately 0.0075 mg/kg, approximately 0.008 mg/kg, approximately 0.009 mg/kg, approximately 0.01 mg/kg, approximately 0.02 mg/kg, approximately 0.025 mg/kg, approximately 0.03 mg/kg, approximately 0.04 mg/kg, approximately 0.05 mg/kg, approximately 0.06 mg/kg, approximately 0.07 mg/kg, approximately 0.075 mg/kg, approximately 0.08 mg/kg, approximately 0.09 mg/kg, approximately 0.1 mg/kg, approximately 0.2 mg/kg, approximately 0.25 mg/kg, approximately 0.3 mg/kg, approximately 0.4 mg/kg, approximately 0.5 mg/kg, approximately 0.6 mg/kg, approximately 0.7 mg/kg, approximately 0.75 mg/kg, approximately 0.8 mg/kg, approximately 0.9 mg/kg, approximately 1 mg/kg, approximately 2 mg/kg, approximately 2.5 mg/kg, approximately 3 mg/kg, approximately 4 mg/kg, approximately 5 mg/kg, approximately 6 mg/kg, approximately 7 mg/kg, approximately 7.5 mg/kg, approximately 8 mg/kg, approximately 9 mg/kg, approximately 10 mg/kg, approximately 11 mg/kg, approximately 12 mg/kg, approximately 12.5 mg/kg, approximately 13 mg/kg, approximately 14 mg/kg, approximately 15 mg/kg, approximately 16 mg/kg, approximately 17 mg/kg, approximately 17.5 mg/kg, approximately 18 mg/kg, approximately 19 mg/kg, approximately 20 mg/kg, approximately 21 mg/kg, approximately 22 mg/kg, approximately 22.5 mg/kg, approximately 23 mg/kg, approximately 24 mg/kg, approximately 25 mg/kg, approximately 26 mg/kg, approximately 27 mg/kg, approximately 27.5 mg/kg, approximately 28 mg/kg, approximately 29 mg/kg, approximately 30 mg/kg, approximately 35 mg/kg, approximately 40 mg/kg, approximately 45 mg/kg, approximately 50 mg/kg, approximately 60 mg/kg, approximately 70 mg/kg, approximately 75 mg/kg, approximately 80 mg/kg, approximately 90 mg/kg, or approximately 100 mg/kg.

"Continued dose" refers to, for example, the dose that is administered continually after administration of the initial dose. The continued dose is nearly the same as or less than the initial dose, and for example, it is nearly the same as the initial dose, or approximately 1/2, approximately 1/3, approximately 1/4, approximately 1/5, approximately 1/6, approximately 1/7, approximately 1/8, approximately 1/9, approximately 1/10, approximately 1/20, approximately 1/25, approximately 1/30, approximately 1/40, approximately 1/50, approximately 1/60, approximately 1/70, approximately 1/80, approximately 1/90, approximately 1/100 of the initial dose, approximately 0.01 times, approximately 0.02 times, approximately 0.03 times, approximately 0.04 times, approximately 0.05 times, approximately 0.06 times, approximately 0.07 times, approximately 0.08 times, approximately 0.09 times, approximately 0.1 times, approximately 0.2 times, approximately 0.25 times, approximately 0.3 times, approximately 0.4 times, approximately 0.5 times, approximately 0.6 times, approximately 0.7 times, approximately 0.8 times, approximately 0.9 times, or approximately 1 time the initial dose.

As long as the continued dose is nearly the same or less than the initial dose, the dose may vary within that range, and the multiple doses during continued dosing do not have to be the same dose. For example, the dose may be decreased gradually, or various doses may arbitrarily be administered repeatedly.

Preferably, the initial dose is 10 mg/kg and at least one of the continued doses is 10 mg/kg, the initial dose is 10 mg/kg and at least one of the continued doses is 3 mg/kg, the initial dose is 10 mg/kg and at least one of the continued doses is 1 mg/kg, the initial dose is 10 mg/kg and at least one of the continued doses is 0.3 mg/kg, the initial dose is 10 mg/kg and at least one of the continued doses is 0.1 mg/kg, the initial dose is 3 mg/kg and at least one of the continued doses is 3 mg/kg, the initial dose is 3 mg/kg and at least one of the continued doses is 1 mg/kg, the initial dose is 3 mg/kg and at least one of the continued doses is 0.3 mg/kg, the initial dose is 3 mg/kg and at least one of the continued doses is 0.1 mg/kg, the initial dose is 1 mg/kg and at least one of the continued doses is 1 mg/kg, the initial dose is 1 mg/kg and at least one of the continued doses is 0.3 mg/kg, or the initial dose is 1 mg/kg and at least one of the continued doses is 0.1 mg/kg.

More preferably, the initial dose is 10 mg/kg and the continued dose is 10 mg/kg, the initial dose is 10 mg/kg and the continued dose is 3 mg/kg, the initial dose is 10 mg/kg and the continued dose is 1 mg/kg, the initial dose is 10 mg/kg and the continued dose is 0.3 mg/kg, the initial dose is 10 mg/kg and the continued dose is 0.1 mg/kg, the initial dose is 3 mg/kg and the continued dose is 3 mg/kg, the initial dose is 3 mg/kg and the continued dose is 1 mg/kg, the initial dose is 3 mg/kg and the continued dose is 0.3 mg/kg, the initial dose is 3 mg/kg and the continued dose is 0.1 mg/kg, the initial dose is 1 mg/kg and the continued dose is 1 mg/kg, the initial dose is 1 mg/kg and the continued dose is 0.3 mg/kg, or the initial dose is 1 mg/kg and the continued dose is 0.1 mg/kg.

Besides mg/kg, the dose may be denoted as a fixed dose (mg/body) and/or a body surface area-based dose (mg/m²) corresponding to the aforementioned body weight-based dose.

The number of times the continued dose is continually administered is not particularly limited, and the number is for example once, twice, three times, four times, five times, six times, seven times, eight times, nine times, ten times, 15 times, 20 times, 25 times, 35 times, 40 times, 50 times, 60 times, 70 times, 80 times, 90 times, 100 times, 500 times, 1000 times, and 10000 times.

"Administration interval" (an interval between individual administrations) indicates the interval between administration of the initial dose and administration of the first continued dose, and the interval between administration of the n^{th} continued dose (n is an integer of 1 or greater) and administration of the (n+1)^{th} continued dose. The administration interval may be one or more days, for example, 2 days, 3 days, 4 days, 5 days, 6 days, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 1 month, 2 months, 3 months, 4 months, 5 months, 6 months, 7 months, 8 months, 9 months, 10 months, 11 months, or 1 year. The administration interval can be expressed in different terms, such as once a day, once every 2 days, once every 3 days, once every 4 days, once every 5 days, once every 6 days, once a week, once every 2 weeks, once every 3 weeks, once every 4 weeks, once every 5 weeks, once every 6 weeks, once every 7 weeks, once every 8 weeks, once every 9 weeks, once every 10 weeks, once every 11 weeks, once every 12 weeks, once every 13 weeks, once every 14 weeks, once every 15 weeks, once every 16 weeks, once every 17 weeks, once every 18 weeks, once every 19 weeks, once every 20 weeks, once every 21 weeks, once every 22 weeks, once every 23 weeks, once every 24 weeks, once every 25 weeks, once a month, once every 2 months, once every 3 months, once every 4 months, once every 5 months, once every 6 months, once every 7 months, once every 8 months, once every 9 months, once every 10 months, once every 11 months, or once a year. Further, the administration interval can be expressed in other terms, such as every day, every 2 days, every 3 days, every 4 days, every 5 days, every 6 days, every week, every 2 weeks, every 3 weeks, every 4 weeks, every 5 weeks, every 6 weeks, every 7 weeks, every 8 weeks, every 9 weeks, every 10 weeks, every 11 weeks, every 12 weeks, every 13 weeks, every 14 weeks, every 15 weeks, every 16 weeks, every 17 weeks, every 18 weeks, every 19 weeks, every 20 weeks, every 21 weeks, every 22 weeks, every 23 weeks, every 24 weeks, every 25 weeks, every month, every 2 months, every 3 months, every 4 months, every 5 months, every 6 months, every 7 months, every 8 months, every 9 months, every 10 months, every 11 months, or every year.

The interval between administration of the initial dose and administration of the first continued dose and the interval between administration of the n^{th} continued dose (n is an integer of 1 or greater) and administration of the (n+1)^{th} continued dose may all be the same; however, they do not have to be all the same. For example, the interval between administration of the initial dose and administration of the first continued dose may be longer than the interval between administration of the n^{th} continued dose and administration of the (n+1)^{th} continued dose; or the interval between administration of the initial dose and administration of the first continued dose may be shorter than the interval between administration of the n^{th} continued dose and administration of the (n+1)^{th} continued dose. Furthermore, the interval between administration of the n^{th} continued dose and administration of the (n+1)^{th} continued dose and the interval between administration of the m^{th} continued dose (m is an integer of 1 or greater) and administration of the (m+1)^{th} continued dose may be the same or different. For example, as the number of times the continued dose is administered increases, the administration interval may become longer or shorter.

Preferably, the initial dose is 10 mg/kg and at least one of the continued doses is 10 mg/kg and the administration interval is one week, the initial dose is 10 mg/kg and at least one of the continued doses is 3 mg/kg and the administration interval is one week, the initial dose is 10 mg/kg and at least one of the continued doses is 1 mg/kg and the administration interval is one week, the initial dose is 10 mg/kg and at least one of the continued doses is 0.3 mg/kg and the administration interval is one week, the initial dose is 10 mg/kg and at least one of the continued doses is 0.1 mg/kg and the administration interval is one week, the initial dose is 3 mg/kg and at least one of the continued doses is 3 mg/kg and the administration interval is one week, the initial dose is 3 mg/kg and at least one of the continued doses is 1 mg/kg and the administration interval is one week, the initial dose is 3 mg/kg and at least one of the continued doses is 0.3 mg/kg and the administration interval is one week, the initial dose is 3 mg/kg and at least one of the continued doses is 0.1 mg/kg and the administration interval is one week, the initial dose is 1 mg/kg and at least one of the continued doses is 1 mg/kg and the administration interval is one week, the initial dose is 1 mg/kg and at least one of the continued doses is 0.3 mg/kg and the administration interval is one week, the initial dose is 1 mg/kg and at least one of the continued doses is 0.1 mg/kg and the administration interval is one week, the initial dose is 10 mg/kg and at least one of the continued doses is 10 mg/kg and the administration interval is 2 weeks, the initial dose is 10 mg/kg and at least one of the continued doses is 3 mg/kg and the administration interval is 2 weeks, the initial dose is 10 mg/kg and at least one of the continued doses is 1 mg/kg and the administration interval is 2 weeks, the initial dose is 10 mg/kg and at least one of the continued doses is 0.3 mg/kg and the administration interval is 2 weeks, the initial dose is 10 mg/kg and at least one of the continued doses is 0.1 mg/kg and the administration interval is 2 weeks, the initial dose is 3 mg/kg and at least one of the continued doses is 3 mg/kg and the administration interval is 2 weeks, the initial dose is 3 mg/kg and at least one of the continued doses is 1 mg/kg and the administration interval is 2 weeks, the initial dose is 3 mg/kg and at least one of the continued doses is 0.3 mg/kg and the administration interval is 2 weeks, the initial dose is 3 mg/kg and at least one of the continued doses is 0.1 mg/kg and the administration interval is 2 weeks, the initial dose is 1 mg/kg and at least one of the continued doses is 1 mg/kg and the administration interval is 2 weeks, the initial dose is 1 mg/kg and at least one of the continued doses is 0.3 mg/kg and the administration interval is 2 weeks, the initial dose is 1 mg/kg and at least one of the continued doses is 0.1 mg/kg and the administration interval is 2 weeks, the initial dose is 10 mg/kg and at least one of the continued doses is 10 mg/kg and the administration interval is 3 weeks, the initial dose is 10 mg/kg and at least one of the continued doses is 3 mg/kg and the administration interval is 3 weeks, the initial dose is 10 mg/kg and at least one of the continued doses is 1 mg/kg and the administration interval is 3 weeks, the initial dose is 10 mg/kg and at least one of the continued doses is 0.3 mg/kg and the administration interval is 3 weeks, the initial dose is 10 mg/kg and at least one of the continued doses is 0.1 mg/kg and the administration interval is 3 weeks, the initial dose is 3 mg/kg and at least one of the continued doses is 3 mg/kg and the administration interval is 3 weeks, the initial dose is 3 mg/kg and at least one of the continued doses is 1 mg/kg and the administration interval is 3 weeks, the initial dose is 3 mg/kg and at least one of the continued doses is 0.3 mg/kg and the administration interval is 3 weeks, the initial dose is 3 mg/kg and at least one of the continued doses is 0.1 mg/kg and the administration interval is 3 weeks, the initial dose is 1 mg/kg and at least one of the continued doses is 1 mg/kg and the administration interval is 3 weeks, the initial dose is 1 mg/kg and at least one of the continued doses is 0.3 mg/kg and the administration interval is 3 weeks, the initial dose is 1 mg/kg and at least one of the continued doses is 0.1 mg/kg and the administration interval is 3 weeks, the initial dose is 10 mg/kg and at least one of the continued doses is 10 mg/kg and the administration interval is 4 weeks, the initial dose is 10 mg/kg and at least one of the continued doses is 3 mg/kg and the administration interval is 4 weeks, the initial dose is 10 mg/kg and at least one of the continued doses is 1 mg/kg and the administration interval is 4 weeks, the initial dose is 10 mg/kg and at least one of the continued doses is 0.3 mg/kg and the administration interval is 4 weeks, the initial dose is 10 mg/kg and at least one of the continued doses is 0.1 mg/kg and the administration interval is 4 weeks, the initial dose is 3 mg/kg and at least one of the continued doses is 3 mg/kg and the administration interval is 4 weeks, the initial dose is 3 mg/kg and at least one of the continued doses is 1 mg/kg and the administration interval is 4 weeks, the initial dose is 3 mg/kg and at least one of the continued doses is 0.3 mg/kg and the administration interval is 4 weeks, the initial dose is 3 mg/kg and at least one of the continued doses is 0.1 mg/kg and the administration interval is 4 weeks, the initial dose is 1 mg/kg and at least one of the continued doses is 1 mg/kg and the administration interval is 4 weeks, the initial dose is 1 mg/kg and at least one of the continued doses is 0.3 mg/kg and the administration interval is 4 weeks, the initial dose is 1 mg/kg and at least one of the continued doses is 0.1 mg/kg and the administration interval is 4 weeks, the initial dose is 10 mg/kg and at least one of the continued doses is 10 mg/kg and the administration interval is 1 month, the initial dose is 10 mg/kg and at least one of the continued doses is 3 mg/kg and the administration interval is 1 month, the initial dose is 10 mg/kg and at least one of the continued doses is 1 mg/kg and the administration interval is 1 month, the initial dose is 10 mg/kg and at least one of the continued doses is 0.3 mg/kg and the administration interval is 1 month, the initial dose is 10 mg/kg and at least one of the continued doses is 0.1 mg/kg and the administration interval is 1 month, the initial dose is 3 mg/kg and at least one of the continued doses is 3 mg/kg and the administration interval is 1 month, the initial dose is 3 mg/kg and at least one of the continued doses is 1 mg/kg and the administration interval is 1 month, the initial dose is 3 mg/kg and at least one of the continued doses is 0.3 mg/kg and the administration interval is 1 month, the initial dose is 3 mg/kg and at least one of the continued doses is 0.1 mg/kg and the administration interval is 1 month, the initial dose is 1 mg/kg and at least one of the continued doses is 1 mg/kg and the administration interval is 1 month, the initial dose is 1 mg/kg and at least one of the continued doses is 0.3 mg/kg and the administration interval is 1 month, the initial dose is 1 mg/kg and at least one of the continued doses is 0.1 mg/kg and the administration interval is 1 month, the initial dose is 10 mg/kg and at least one of the continued doses is 10 mg/kg and the administration interval is 2 months, the initial dose is 10 mg/kg and at least one of the continued doses is 3 mg/kg and the administration interval is 2 months, the initial dose is 10 mg/kg and at least one of the continued doses is 1 mg/kg and the administration interval is 2 months, the initial dose is 10 mg/kg and at least one of the continued doses is 0.3 mg/kg and the administration interval is 2 months, the initial dose is 10 mg/kg and at least one of the continued doses is 0.1 mg/kg and the administration interval is 2 months, the initial dose is 3 mg/kg and at least one of the continued doses is 3 mg/kg and the administration interval is 2 months, the initial dose is 3 mg/kg and at least one of the continued doses is 1 mg/kg and the administration interval is 2 months, the initial dose is 3 mg/kg and at least one of the continued doses is 0.3 mg/kg and the administration interval is 2 months, the initial dose is 3 mg/kg and at least one of the continued doses is 0.1 mg/kg and the administration interval is 2 months, the initial dose is 1 mg/kg and at least one of the continued doses is 1 mg/kg and the administration interval is 2 months, the initial dose is 1 mg/kg and at least one of the continued doses is 0.3 mg/kg and the administration interval is 2 months, or the initial dose is 1 mg/kg and at least one of the continued doses is 0.1 mg/kg and the administration interval is 2 months.

More preferably, the initial dose is 10 mg/kg and the continued dose is 10 mg/kg and the administration interval is one week, the initial dose is 10 mg/kg and the continued dose is 3 mg/kg and the administration interval is one week, the initial dose is 10 mg/kg and the continued dose is 1 mg/kg and the administration interval is one week, the initial dose is 10 mg/kg and the continued dose is 0.3 mg/kg and the administration interval is one week, the initial dose is 10 mg/kg and the continued dose is 0.1 mg/kg and the administration interval is one week, the initial dose is 3 mg/kg and the continued dose is 3 mg/kg and the administration interval is one week, the initial dose is 3 mg/kg and the continued dose is 1 mg/kg and the administration interval is one week, the initial dose is 3 mg/kg and the continued dose is 0.3 mg/kg and the administration interval is one week, the initial dose is 3 mg/kg and the continued dose is 0.1 mg/kg and the administration interval is one week, the initial dose is 1 mg/kg and the continued dose is 1 mg/kg and the administration interval is one week, the initial dose is 1 mg/kg and the continued dose is 0.3 mg/kg and the administration interval is one week, the initial dose is 1 mg/kg and the continued dose is 0.1 mg/kg and the administration interval is one week, the initial dose is 10 mg/kg and the continued dose is 10 mg/kg and the administration interval is 2 weeks, the initial dose is 10 mg/kg and the continued dose is 3 mg/kg and the administration interval is 2 weeks, the initial dose is 10 mg/kg and the continued dose is 1 mg/kg and the administration interval is 2 weeks, the initial dose is 10 mg/kg and the continued dose is 0.3 mg/kg and the administration interval is 2 weeks, the initial dose is 10 mg/kg and the continued dose is 0.1 mg/kg and the administration interval is 2 weeks, the initial dose is 3 mg/kg and the continued dose is 3 mg/kg and the administration interval is 2 weeks, the initial dose is 3 mg/kg and the continued dose is 1 mg/kg and the administration interval is 2 weeks, the initial dose is 3 mg/kg and the continued dose is 0.3 mg/kg and the administration interval is 2 weeks, the initial dose is 3 mg/kg and the continued dose is 0.1 mg/kg and the administration interval is 2 weeks, the initial dose is 1 mg/kg and the continued dose is 1 mg/kg and the administration interval is 2 weeks, the initial dose is 1 mg/kg and the continued dose is 0.3 mg/kg and the administration interval is 2 weeks, the initial dose is 1 mg/kg and the continued dose is 0.1 mg/kg and the administration interval is 2 weeks, the initial dose is 10 mg/kg and the continued dose is 10 mg/kg and the administration interval is 3 weeks, the initial dose is 10 mg/kg and the continued dose is 3 mg/kg and the administration interval is 3 weeks, the initial dose is 10 mg/kg and the continued dose is 1 mg/kg and the administration interval is 3 weeks, the initial dose is 10 mg/kg and the continued dose is 0.3 mg/kg and the administration interval is 3 weeks, the initial dose is 10 mg/kg and the continued dose is 0.1 mg/kg and the administration interval is 3 weeks, the initial dose is 3 mg/kg and the continued dose is 3 mg/kg and the administration interval is 3 weeks, the initial dose is 3 mg/kg and the continued dose is 1 mg/kg and the administration interval is 3 weeks, the initial dose is 3 mg/kg and the continued dose is 0.3 mg/kg and the administration interval is 3 weeks, the initial dose is 3 mg/kg and the continued dose is 0.1 mg/kg and the administration interval is 3 weeks, the initial dose is 1 mg/kg and the continued dose is 1 mg/kg and the administration interval is 3 weeks, the initial dose is 1 mg/kg and the continued dose is 0.3 mg/kg and the administration interval is 3 weeks, the initial dose is 1 mg/kg and the continued dose is 0.1 mg/kg and the administration interval is 3 weeks, the initial dose is 10 mg/kg and the continued dose is 10 mg/kg and the administration interval is 4 weeks, the initial dose is 10 mg/kg and the continued dose is 3 mg/kg and the administration interval is 4 weeks, the initial dose is 10 mg/kg and the continued dose is 1 mg/kg and the administration interval is 4 weeks, the initial dose is 10 mg/kg and the continued dose is 0.3 mg/kg and the administration interval is 4 weeks, the initial dose is 10 mg/kg and the continued dose is 0.1 mg/kg and the administration interval is 4 weeks, the initial dose is 3 mg/kg and the continued dose is 3 mg/kg and the administration interval is 4 weeks, the initial dose is 3 mg/kg and the continued dose is 1 mg/kg and the administration interval is 4 weeks, the initial dose is 3 mg/kg and the continued dose is 0.3 mg/kg and the administration interval is 4 weeks, the initial dose is 3 mg/kg and the continued dose is 0.1 mg/kg and the administration interval is 4 weeks, the initial dose is 1 mg/kg and the continued dose is 1 mg/kg and the administration interval is 4 weeks, the initial dose is 1 mg/kg and the continued dose is 0.3 mg/kg and the administration interval is 4 weeks, the initial dose is 1 mg/kg and the continued dose is 0.1 mg/kg and the administration interval is 4 weeks, the initial dose is 10 mg/kg and the continued dose is 10 mg/kg and the administration interval is 1 month, the initial dose is 10 mg/kg and the continued dose is 3 mg/kg and the administration interval is 1 month, the initial dose is 10 mg/kg and the continued dose is 1 mg/kg and the administration interval is 1 month, the initial dose is 10 mg/kg and the continued dose is 0.3 mg/kg and the administration interval is 1 month, the initial dose is 10 mg/kg and the continued dose is 0.1 mg/kg and the administration interval is 1 month, the initial dose is 3 mg/kg and the continued dose is 3 mg/kg and the administration interval is 1 month, the initial dose is 3 mg/kg and the continued dose is 1 mg/kg and the administration interval is 1 month, the initial dose is 3 mg/kg and the continued dose is 0.3 mg/kg and the administration interval is 1 month, the initial dose is 3 mg/kg and the continued dose is 0.1 mg/kg and the administration interval is 1 month, the initial dose is 1 mg/kg and the continued dose is 1 mg/kg and the administration interval is 1 month, the initial dose is 1 mg/kg and the continued dose is 0.3 mg/kg and the administration interval is 1 month, the initial dose is 1 mg/kg and the continued dose is 0.1 mg/kg and the administration interval is 1 month, the initial dose is 10 mg/kg and the continued dose is 10 mg/kg and the administration interval is 2 months, the initial dose is 10 mg/kg and the continued dose is 3 mg/kg and the administration interval is 2 months, the initial dose is 10 mg/kg and the continued dose is 1 mg/kg and the administration interval is 2 months, the initial dose is 10 mg/kg and the continued dose is 0.3 mg/kg and the administration interval is 2 months, the initial dose is 10 mg/kg and the continued dose is 0.1 mg/kg and the administration interval is 2 months, the initial dose is 3 mg/kg and the continued dose is 3 mg/kg and the administration interval is 2 months, the initial dose is 3 mg/kg and the continued dose is 1 mg/kg and the administration interval is 2 months, the initial dose is 3 mg/kg and the continued dose is 0.3 mg/kg and the administration interval is 2 months, the initial dose is 3 mg/kg and the continued dose is 0.1 mg/kg and the administration interval is 2 months, the initial dose is 1 mg/kg and the continued dose is 1 mg/kg and the administration interval is 2 months, the initial dose is 1 mg/kg and the continued dose is 0.3 mg/kg and the administration interval is 2 months, or the initial dose is 1 mg/kg and the continued dose is 0.1 mg/kg and the administration interval is 2 months.

The dosage regimen is determined, for example, by considering the effects and safety. Furthermore, the dosage regimen is determined by considering the convenience of the patient, within the range that does not impair the effectiveness and safety. For example, the dosage regimen for a hemophilia A patient can be determined by considering the effects of preventing bleeding in patients and clinically acceptable safety.

In the present invention, "nearly the same" means that the difference is approximately 20% or less, preferably the difference is 10% or less, or more preferably the difference is 5% or less, 4% or less, 3% or less, 2% or less, or 1% or less.

A disease that develops and/or progresses due to a decrease or deficiency in the activity of blood coagulation factor VIII and/or activated blood coagulation factor VIII (FVIIIa) is, for example, hemophilia A, hemophilia A with emergence of an inhibitor against FVIII / FVIIIa, acquired hemophilia A, and von Willebrand disease, but the disease is not particularly limited thereto.

The present invention provides a product comprising at least (i) a container; (ii) a pharmaceutical composition in a container, which comprises a bispecific antigen-binding molecule that recognizes blood coagulation factor IX and/or activated blood coagulation factor IX and blood coagulation factor X and/or activated blood coagulation factor X; and (iii) a document instructing administration of the antigen-binding molecule at an initial dose of approximately 0.001 to 100 mg/kg and multiple continued administrations of continued doses that are nearly the same as or less than the initial dose with administration intervals of at least one day or longer. In addition, a label, syringe, syringe needle, pharmaceutically acceptable medium, alcohol-soaked cotton, adhesive bandage, and such may be packaged in the product. The container is, for example, a bottle, a glass bottle, or a syringe, and can be produced from various materials such as glass or plastic. A pharmaceutical composition is stored in the container, and the mouth of the container is sealed with a rubber stopper or such. For example, a label indicating that the pharmaceutical composition is to be used for preventing or treating selected pathological conditions is attached to the container.

Treatment of hemophilia refers to, for example, stopping bleeding by administering the composition to a hemophilia patient who is actually showing bleeding symptoms (treatment of bleeding) and/or reducing the bleeding frequency by administering the composition to a patient who had shown bleeding to prevent manifestation of bleeding symptoms in advance (prevention of bleeding), but it is not limited thereto. Treatment and prevention of bleeding may be understood as having the same meaning in certain cases.

Herein, bleeding that is examined and counted as the number of bleeding events in a patient is, for example, bleeding that required hemostatic treatment by coagulation factor formulations. Coagulation factor formulations refer to, for example, FVIII formulations and bypassing agents (activated prothrombin complex formulations, recombinant FVIIa formulations, and such).

The number of bleedings per year (the Annualized Bleeding Rate (ABR)) is calculated as: (number of bleeding events x 365.25) / number of days of observation.

All prior art references cited herein are incorporated by reference into this description.

### Examples

Herein below, the present invention will be specifically described with reference to the Examples, but it is not to be construed as being limited thereto.

### Example 1: Preparation of a bispecific antibody that recognizes blood coagulation factor IX and/or activated blood coagulation factor IX and blood coagulation factor X and/or activated blood coagulation factor X

ACE910 (Q499-z121/J327-z119/L404-k) which is a bispecific antibody described in a non-patent document (PLoS One. 2013;8(2):e57479) and a patent document (WO 2012/067176) (a bispecific antibody in which the H chain containing the amino acid sequence of SEQ ID NO: 20 and the L chain of SEQ ID NO: 32 are associated, and the H chain containing the amino acid sequence of SEQ ID NO: 25 and the L chain of SEQ ID NO: 32 are associated) was produced according to description in the aforementioned non-patent document or patent document. As described in the patent document, ACE910 has an activity that substitutes for the function of coagulation factor VIII.

### Example 2: Single subcutaneous administration study in healthy adult Japanese and Caucasian males

A single dose of ACE910 at a volume (µL/kg) shown in Table 1 was subcutaneously administered to the abdomen in the test drug groups of Part A (Japanese) and Part B (Caucasians). ACE910 administration solutions were prepared at concentrations (mg/mL) shown in the table below, and administered at the indicated volumes (µL/kg). For administration of 0.1 mg/kg or less, a diluent (prepared by diluting an auxiliary diluent approximately 100-times with a physiological saline solution) was used; and for administration of 0.3 mg/kg, a physiological saline solution was used.

**Table 1**

| Examination | ACE910 dose (mg/kg) | ACE910 dose solution concentration (mg/mL) | Dose volume (µL/kg) |
|---|---|---|---|
| A-1 | 0.001 | 0.08 | 12.5 |
| A-2 | 0.01 | 0.8 | 12.5 |
| A-3, B-1 | 0.1 | 8 | 12.5 |
| A-4, B-2 | 0.3 | 24 | 12.5 |
| A-5, B-3 | 1 | 80 | 12.5 |

Follow-up observations were performed for 4 weeks (A-1, A-2), 16 weeks (A-3, B-1), 20 weeks (A-4, B-2), and 24 weeks (A-5, B-3) after administration, and serious side effects were not particularly observed.

### Example 3: Open-label, inter-individual, dose-ascending, multiple subcutaneous administration study in Japanese hemophilia A patients (Examinations 1 and 2)

Patients meeting the following criteria were selected as the study participants:
(1) a patient with severe congenital hemophilia A;
(2) a Japanese individual;
(3) an individual whose body weight is 40 kg or more;
(4) an individual whose record of bleeding episodes (bleeding period, bleeding site) and treatment with blood coagulation factor formulations is available for the six months prior to enrollment; and
(5) an individual who meets either one of the following criteria at the time of enrollment:
   (a) for a patient having inhibitors, an individual who has had six or more bleeding events during the six months prior to enrollment; and
   (b) for a patient not having inhibitors, an individual who has received 150 or more administrations of FVIII formulations before enrollment and has been carrying out regular replacement therapy with FVIII formulations for the six months prior to enrollment.

Based on the latest body weight measurement, the dose volume ofACE910 shown in Table 2 was repeatedly administered subcutaneously to the abdomen at a frequency of once a week for 12 weeks (total of 12 times). The test drug used was 1.0 mL of a solution containing 80 mg of ACE910 in a single vial.

**Table 2**

| Dose, dose solution concentration, and dose volume of ACE910 | | | | | | |
|---|---|---|---|---|---|---|
| Examination | Initial administration | | | 2nd and subsequent administrations | | |
| | ACE910 dose (mg/kg) | ACE910 dose solution concentra t ion (mg/mL) | Dose volume (µL/kg) | AC E 910 dose (mg/kg) | ACE910 dose solution concentra tion (mg/mL) | Dose volume (µL/kg) |
| 1 | 1 | 80 | 12.5 | 0.3 | 24 | 12.5 |
| 2 | 3 | 80 | 37.5 | 1 | 80 | 12.5 |

To administer 0.3 mg/kg, the 80-mg/mL ACE910-containing solution was adjusted to have the ACE910 administration solution concentration (mg/mL) shown in Table 2 using a physiological saline solution, and then this was administered at the administration volume (µL/kg) shown in Table 2. The maximum volume that can be administered per administration site was set to 1.5 mL, and when a larger volume was to be administered, subcutaneous administrations were carried out at two or more separate sites. During the period of ACE910 administration, regular replacement therapy with FVIII formulations was discontinued.

The number of bleeding events for the six months prior to ACE910 administration, and the number of bleeding events during the ACE910 administration period (12 weeks) were investigated. Bleeding events that required hemostatic therapy with FVIII formulations or bypassing agents were investigated. The numbers of bleeding events before and after ACE910 administration were converted to annualized bleeding frequencies. Specifically, the annualized bleeding rate (ABR) was calculated as: (number of bleeding events x 365.25) / number of days of observation.

An interim analysis of Examination 1 showed that inhibitor patients who have not been given any prophylactic treatment against bleeding showed a dramatic decrease in the annualized bleeding frequency by ACE910 administration in comparison to when ACE910 was not administered. Moreover, in non-inhibitor patients who had been given regular replacement therapy with FVIII formulations, the annualized bleeding frequency was decreased by ACE910 administration in comparison to when FVIII formulations were administered (Fig. 1).

Examination 2 which was carried out with changing the ACE910 dose showed a dramatic decrease in the annualized bleeding frequency by ACE910 administration in both inhibitor patients who had not been given any prophylactic treatment against bleeding and non-inhibitor patients who had been given regular replacement therapy with FVIII formulations (Fig. 2).

Since ACE910 has a long half-life in blood, its administration frequency is low, it can be administered subcutaneously, it is effective also for inhibitor patients, it may not induce inhibitors, and such; therefore, it is considered to be a prominent pharmaceutical agent.

### Example 4: Open-label, inter-individual, dose-ascending, multiple subcutaneous administration study in Japanese hemophilia A patients (Examination 3)

Patients were selected according to the same criteria as in Example 3, Examination 3 described in Table 3 was performed, and the annualized bleeding rate was investigated.

Based on the latest body weight measurement, the dose volume of ACE910 shown in Table 3 was repeatedly administered subcutaneously to the abdomen at a frequency of once a week for 12 weeks (total of 12 times). The test drug used was 1.0 mL of a solution containing 80 mg of ACE910 in a single vial.

**Table 3**

| Dose, dose solution concentration, and dose volume of ACE910 | | | | | | |
|---|---|---|---|---|---|---|
| Examination | Initial administration | | | 2nd and subsequent administrations | | |
| | ACE910 dose (mg/kg) | ACE910 dose solution concentration (mg/mL) | Dose volume (µL/kg) | ACE910 dose (mg/kg) | ACE910 dose solution concentration (mg/mL) | Dose volume (µL/kg) |
| 3 | 3 | 80 | 37.5 | 3 | 80 | 37.5 |

Preparation of the administration solution, investigation of the bleeding rate, and such were carried out according to Example 3. Regarding Examination 3, the annualized bleeding frequency was dramatically decreased by ACE910 administration in both inhibitor patients who had been given regular administration therapy with bypassing agents that prevent manifestation of bleeding (for 6 months prior to ACE910 administration) and non-inhibitor patients who had been given regular replacement therapy with FVIII formulations (for 6 months prior to ACE910 administration) (Fig. 3).

### Industrial Applicability

The present invention provides a pharmaceutical composition comprising a bispecific antigen-binding molecule which recognizes blood coagulation factor IX and/or activated blood coagulation factor IX and blood coagulation factor X and/or activated blood coagulation factor X, as a more effective pharmaceutical composition for preventing and/or treating a disease which develops and/or progresses due to a decrease or deficiency in the activity of blood coagulation factor VIII and/or activated blood coagulation factor VIII, or a dosage regimen thereof.

Furthermore, the invention relates to the following items:
1. A pharmaceutical composition for use in preventing and/or treating a disease that develops and/or progresses due to a decrease or deficiency in the activity of blood coagulation factor VIII and/or activated blood coagulation factor VIII, wherein the composition comprises a bispecific antigen-binding molecule that recognizes blood coagulation factor IX and/or activated blood coagulation factor IX and blood coagulation factor X and/or activated blood coagulation factor X, wherein the antigen-binding molecule is administered at an initial dose of approximately 0.001 to 100 mg/kg and is continually administered several times at continued doses that are nearly the same as or less than the initial dose, wherein the interval between individual administrations is at least one day or longer.
2. The pharmaceutical composition of item 1, wherein the continued dose is a dose selected from the group consisting of the same dose as the initial dose, and approximately one-half, approximately one-third, approximately 0.3-times, approximately one-fourth, approximately one-fifth, and approximately one-tenth the initial dose.
3. The pharmaceutical composition of item 2, wherein the initial dose is 1 mg/kg and at least one of the continued doses is 0.3 mg/kg.
4. The pharmaceutical composition of item 2, wherein the initial dose is 3 mg/kg and at least one of the continued doses is 1 mg/kg.
5. The pharmaceutical composition of item 2, wherein the initial dose is 3 mg/kg and at least one of the continued doses is 3 mg/kg.
6. The pharmaceutical composition of any one of items 1 to 5, wherein at least one of the intervals between individual administrations is one week.
7. The pharmaceutical composition of any one of items 1 to 6, wherein the disease is selected from the group consisting of hemophilia A, acquired hemophilia A, von Willebrand disease, and hemophilia A with emergence of an inhibitor against blood coagulation factor VIII and/or activated blood coagulation factor VIII.
8. The pharmaceutical composition of any one of items 1 to 7, wherein the antigen-binding molecule is the bispecific antibody described below, wherein the first polypeptide and the third polypeptide are associated and the second polypeptide and the fourth polypeptide are associated:
   a bispecific antibody comprising a first polypeptide which is an H chain comprising the amino acid sequence of SEQ ID NO: 20; a second polypeptide which is an H chain comprising the amino acid sequence of SEQ ID NO: 25; a third polypeptide and a fourth polypeptide which are a commonly shared L chain comprising the amino acid sequence of SEQ ID NO: 32.
9. The pharmaceutical composition of any one of items 1 to 8, wherein the antigen-binding molecule is administered subcutaneously.
10. A product comprising (i) a container; (ii) a pharmaceutical composition in the container, wherein the composition comprises a bispecific antigen-binding molecule that recognizes blood coagulation factor IX and/or activated blood coagulation factor IX and blood coagulation factor X and/or activated blood coagulation factor X; and (iii) a document instructing administration of the antigen-binding molecule at an initial dose of approximately 0.001 to 100 mg/kg and several continued administrations of continued doses that are nearly the same as or less than the initial dose, wherein the interval between individual administrations is at least one day or longer.
11. The product of item 10, wherein the initial dose is 1 mg/kg and at least one of the continued doses is 0.3 mg/kg.
12. The product of item 10 or 11, wherein at least one of the intervals between individual administrations is one week.
13. The product of any one of items 10 to 12, which further comprises a label attached to the container which indicates that the pharmaceutical composition can be used for preventing and/or treating a disease that develops and/or progresses due to a decrease or deficiency in the activity of blood coagulation factor VIII and/or activated blood coagulation factor VIII.
14. The product of item 13, wherein the label indicates that the pharmaceutical composition can be used for the treatment of hemophilia A, acquired hemophilia A, von Willebrand disease, and hemophilia A with emergence of an inhibitor against blood coagulation factor VIII and/or activated blood coagulation factor VIII.
15. The product of any one of items 10 to 14, wherein the antigen-binding molecule is the bispecific antibody described below, wherein the first polypeptide and the third polypeptide are associated and the second polypeptide and the fourth polypeptide are associated:
   a bispecific antibody comprising a first polypeptide which is an H chain comprising the amino acid sequence of SEQ ID NO: 20; a second polypeptide which is an H chain comprising the amino acid sequence of SEQ ID NO: 25; and a third polypeptide and a fourth polypeptide which are commonly shared L chain comprising the amino acid sequence of SEQ ID NO: 32.

## Claims

1. A pharmaceutical composition for use in preventing and/or treating hemophilia A, wherein the composition comprises a bispecific antigen-binding molecule that recognizes blood coagulation factor IX and/or activated blood coagulation factor IX and blood coagulation factor X and/or activated blood coagulation factor X, wherein the bispecific antigen-binding molecule is administered subcutaneously at an initial dose of 3 mg/kg and continually administered subcutaneously several times at continued doses, each of which is the same as the initial dose, wherein the interval between individual subcutaneous administrations is one week, and wherein the bispecific antigen-binding molecule is the bispecific antibody described below, wherein the first polypeptide and the third polypeptide are associated and the second polypeptide and the fourth polypeptide are associated:
a bispecific antibody comprising a first polypeptide which is an H chain comprising the amino acid sequence of SEQ ID NO: 20; a second polypeptide which is an H chain comprising the amino acid sequence of SEQ ID NO: 25; a third polypeptide and a fourth polypeptide which are a commonly shared L chain comprising the amino acid sequence of SEQ ID NO: 32.

2. A product comprising (i) a container with a label attached thereto which indicates use for preventing and/or treating hemophilia A; (ii) a pharmaceutical composition in the container, wherein the composition comprises a bispecific antigen-binding molecule that recognizes blood coagulation factor IX and/or activated blood coagulation factor IX and blood coagulation factor X and/or activated blood coagulation factor X; and (iii) a document instructing subcutaneous administration of the bispecific antigen-binding molecule at an initial dose of 3 mg/kg and several continued subcutaneous administrations of continued doses, each of which is the same as the initial dose, wherein the interval between individual subcutaneous administrations is one week, wherein the pharmaceutical composition is subcutaneously administered according to the instructions in the document, wherein the bispecific antigen-binding molecule is the bispecific antibody described below, wherein the first polypeptide and the third polypeptide are associated and the second polypeptide and the fourth polypeptide are associated:
a bispecific antibody comprising a first polypeptide which is an H chain comprising the amino acid sequence of SEQ ID NO: 20; a second polypeptide which is an H chain comprising the amino acid sequence of SEQ ID NO: 25; and a third polypeptide and a fourth polypeptide which are commonly shared L chain comprising the amino acid sequence of SEQ ID NO: 32.
